# FASCICULE DE BREVET EUROPEEN

(11) **EP 1 519 709 B1**
(45) Date de publication et mention de la délivrance du brevet: **06.04.2011**
(21) Numéro de dépôt: 03763956.4
(22) Date de dépôt: 10.07.2003
(51) Int. Cl.: A61K 8/34, A61K 8/86, A61K 8/97, A61Q 19/02

(54) **NOUVELLES APPLICATIONS COSMETIQUES DE POLYPHENOLS ET DE LEURS DERIVES**
NEUE KOSMETISCHE ANWENDUNGEN VON POLYPHENOLEN UND IHREN DERIVATEN
NOVEL COSMETIC APPLICATIONS OF POLYPHENOLS AND DERIVATIVES THEREOF

(30) Priorité: 10.07.2002 FR 0208694
(43) Date de publication de la demande: 06.04.2005
(73) Titulaire: Caudalie, 75017 Paris (FR)
(72) Inventeur: VERCAUTEREN, Joseph, F-34170 Castelnau-le-Lez (FR); CASTAGNINO, Chantal, F-34000 Montpellier (FR)
(74) Mandataire: Peaucelle, Chantal
(86) Numéro de dépôt international: PCT/FR2003/002181
(87) Numéro de publication internationale: WO 2004/006881

(56) Documents cités:
- WO-A-01/91764
- WO-A-99/03816
- FR-A- 2 795 964
- FR-A- 2 816 843
- DATABASE BIOSIS [en ligne] BIOSCIENCES INFORMATION SERVICE, PHILADELPHIA, PA, US; avril 1998 (1998-04) KANG BO-SEONG ET AL: "Inhibitory effects of alpha-viniferin and resveratrol on the L-dopa oxidase activity of tyrosinase." Database accession no. PREV199800307983 XP002234633 & MEDICAL SCIENCE RESEARCH, vol. 26, no. 4, avril 1998 (1998-04), pages 235-237, ISSN: 0269-8951
- DATABASE MEDLINE [en ligne] US NATIONAL LIBRARY OF MEDICINE (NLM), BETHESDA, MD, US; juillet 2000 (2000-07) BADERSCHNEIDER B ET AL: "Isolation and characterization of novel stilbene derivatives from Riesling wine." Database accession no. NLM11032479 XP002234634 & JOURNAL OF AGRICULTURAL AND FOOD CHEMISTRY. UNITED STATES JUL 2000, vol. 48, no. 7, juillet 2000 (2000-07), pages 2681-2686, ISSN: 0021-8561
- OHYAMA M ET AL: "Antitumor Agents 200. Cytotoxicity of Naturally Occurring Resveratrol Oligomers and their Acetate Derivatives" BIOORGANIC & MEDICINAL CHEMISTRY LETTERS, OXFORD, GB, vol. 9, no. 20, 18 octobre 1999 (1999-10-18), pages 3057-3060, XP004180537 ISSN: 0960-894X
- YAN K-X ET AL: "A novel oligostilbene named (+)-viniferol A from the stem of Vitis vinifera 'Kyohou'" TETRAHEDRON, ELSEVIER SCIENCE PUBLISHERS, AMSTERDAM, NL, vol. 57, no. 14, 2 avril 2001 (2001-04-02), pages 2711-2715, XP004232230 ISSN: 0040-4020
- TANAKA T ET AL: "A resveratrol dimer from Parthenocissus tricuspidata" PHYTOCHEMISTRY, PERGAMON PRESS, GB, vol. 48, no. 7, août 1998 (1998-08), pages 1241-1243, XP004289956 ISSN: 0031-9422
- DATABASE MEDLINE [en ligne] US NATIONAL LIBRARY OF MEDICINE (NLM), BETHESDA, MD, US; juin 2002 (2002-06) KIM HYO JIN ET AL: "Cytotoxic and antimutagenic stilbenes from seeds of Paeonia lactiflora." Database accession no. NLM12135100 XP002234635 & ARCHIVES OF PHARMACAL RESEARCH. KOREA (SOUTH) JUN 2002, vol. 25, no. 3, juin 2002 (2002-06), pages 293-299, ISSN: 0253-6269
- DATABASE BIOSIS [en ligne] BIOSCIENCES INFORMATION SERVICE, PHILADELPHIA, PA, US; 27 février 2002 (2002-02-27) PRIVAT CHRISTELLE ET AL: "Antioxidant properties of trans-epsilon-viniferin as compared to stilbene derivatives in aqueous and nonaqueous media." Database accession no. PREV200200208775 XP002234636 & JOURNAL OF AGRICULTURAL AND FOOD CHEMISTRY, vol. 50, no. 5, 27 février 2002 (2002-02-27), pages 1213-1217, February 27, 2002 ISSN: 0021-8561

## Description

L'invention a pour objet de nouvelles applications cosmétiques de polyphénols et de leurs dérivés.

Divers travaux ont porté ces dernières années sur les propriétés anti-radicaux libres des polyphénols et sur leurs applications en thérapeutique et en cosmétique. Plus spécialement, ces travaux ont concerné les oligomères procyanidoliques extraits de végétaux tels que la vigne, le pin ou le thé vert, ou encore du resvératrol, et ses oligomères extraits, en particulier, de la vigne ou encore de polygonacées.

En avril 1998, Kang Bo-Seong et al, rapportaient les effets inhibiteurs de l'α-viniférine et du resvératrol sur l'activité L-Dopa oxydase de la tyrosinase Medical Science Reseach, 26(4), 235-237.

Or, de manière surprenante, les inventeurs ont constaté que cet effet inhibiteur était plus important avec des oligomères et/ou des polymères du resvératrol autres que l'α-viniférine, ou encore avec des mélanges de resvératrol et d'oligomères de resvératrol.

L'invention vise donc l'utilisation d'ε-viniférine, de mélanges d'oligomères et/ou de polymères de resvératrol comprennant de l'ε-viniferine et renfermant le cas échéant du resvératrol monomère, et leurs dérivés de type esters et ethers dans des compositions cosmétiques, comme agents de blanchiment de la peau et/ou agents anti-tâches.

Par le terme "polyphénol", on entend les polyphénols natifs, plus spécialement le resvératrol et ses mélanges avec des oligomères, tels qu'obtenus par exemple par extraction à partir de rafles ou de sarments de vigne. Ce terme englobe aussi les polyphénols dérivatisés de ces composés, en particulier les esters avec des acides gras tels que définis dans le brevet FR n° 97 08 964 du 15 juillet 1997 et les éthers.

L'invention vise plus spécialement l'utilisation cosmétique à des fins de blanchiment de la peau et/ou anti-tâches, de compositions caractérisées en ce qu'elles renferment une quantité efficace pour obtenir les effets ci-dessus de polyphénols choisis dans le groupe comprenant l'ε-viniférine, de mélanges d'oligomères et/ou de polymères de resvératrol, renfermant le cas échéant du resvératrol, comprenant de l'ε-viniférine, et leurs derivés de type esters et éthers. Des mélanges avantageux comprennent du resvératrol monomère et de l'ε-viniférine selon des rapports de 10/90 à 90/10. Les mélanges ci-dessus peuvent en outre comprendre des composés polyphénoliques, en particuliers stilbéniques et/ou flavonoliques, comme les flavanols.

L'invention vise en particulier l'utilisation des compositions renfermant les dérivés de type esters et éthers ci-dessus. De tels dérivés permettent d'améliorer leur bio-disponibilité.

Comme illustré par les exemples, un effet de blanchiment de la peau et/ou anti-tâches des polyphénols et dérivés ci-dessus a été mis en évidence par les inventeurs dans des tests d'inhibition de la tyrosinase. Cet effet est également observé avec l'ε-viniférine seule.

Alors que dans le cas du resvératrol, la CI₅ₒ est supérieure à 60µM dans des tests d'inhibition de tyrosinase, de manière inattendue, les composés et mélanges ci-dessus présentent des CI₅₀ inférieures à 30µM dans les conditions expérimentales rapportées dans les exemples.

Dans l'utilisation selon l'invention, les compositions cosmétiques renferment de 0,1 à 5 % de polyphénols par rapport au poids total de la composition, et un véhicule acceptable en cosmétologie.

Ces compositions cosmétiques se présentent notamment sous forme de crème, pommade, émulsion, liposome, gel, lotion, spray.

Elles peuvent renfermer d'autres principes actifs.

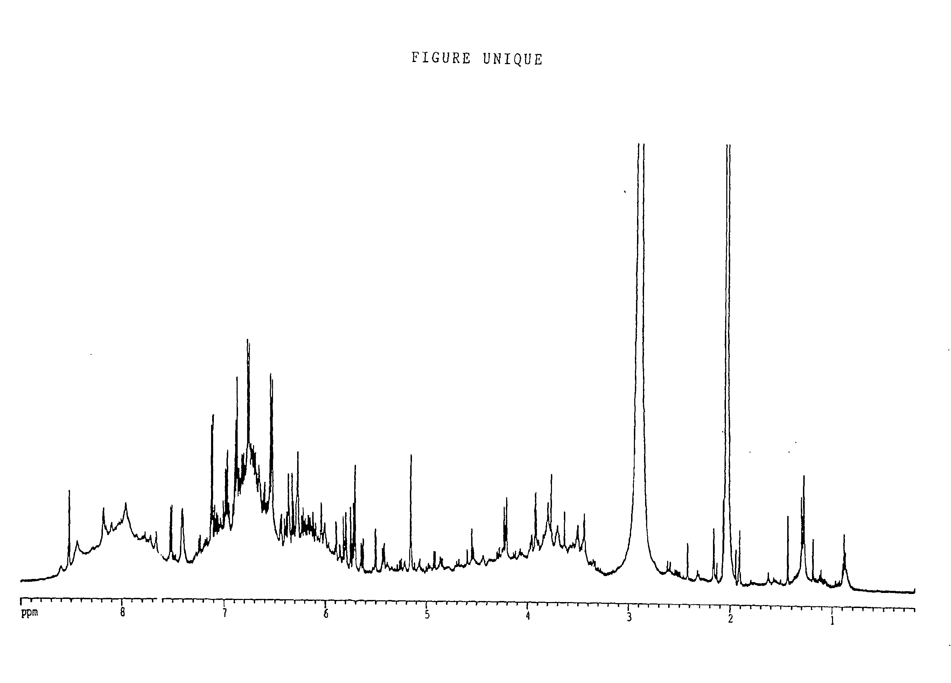

D'autres caractéristiques et avantages de l'invention seront décrits plus en détail ci-après à des fins d'illustration de l'invention, sans caractère limitatif. Dans ces exemples, il est fait référence à la figure unique qui représente le spectre de RMN d'un mélange de polymères (bloc X) selon l'invention.

### Exemple 1 : Etude de l'effet inhibiteur de polyphénols vis-à-vis de la tyrosinase.

### A/ Polyphénols testés

On rapporte les résultats obtenus avec les blocs I à X suivantes :
- Bloc I : extrait polyphénolique total obtenu par extraction à partir de sarments de vigne (extrait "OR")
- Bloc II (comparatif) : resvératrol (pureté d'au moins 95%)
- Bloc III : resvératrol / ε-viniférine (75/25),
- Bloc IV : resvératrol / ε-viniférine (64/36),
- Bloc V : resvératrol / ε-viniférine (55/45),
- Bloc VI : resvératrol / ε-viniférine (50/50),
- Bloc VII : resvératrol / ε-vinifërine (25/75),
- Bloc VIII : ε-viniférine (pureté d'au moins 95%),
- Bloc IX (comparatif) : deux autres dimères (PM 454g/mole) du resvératrol (51 ± 10%)
   + trimère du resvératrol (PM 680g/mole) (33 ± 10%)
   + dimère hydraté du resvératrol (PM 470g/mole) (9 ± 5%)
   + flavanols (290g/mole) (6 ± 5%)
- Bloc X (comparatif) : polymères polyphénoliques

Ces blocs sont obtenus par fractionnement d'extraits polyphénoliques ("OR") préparés à partir de sarments de vigne, par exemple par chromatographie de partage centrifuge (CPC).

Le système de solvants utilisé est le suivant : hexane/acétate d'éthyle/ éthanol/ eau 4/5/3/3, en mode ascendant. Un suivi par chromatographie sur couche mince permet de regrouper les fractions en 4 blocs (II, IV, VIII et IX). Les composés polaires non élués, récupérés lors du rinçage de la colonne, constituent le bloc X (spectre UV de ce bloc : λₘₓ méthanol 223 et 281 nm).

En variante, les mélanges d'oligomères peuvent être obtenus à partir de l'extrait d'OR par chromatographie sur un support solide, comme par exemple la silice, le Séphadex®, ou le Fractogel®.

### B/ Test d'inhibition de la tyrosinase

Mélange A : l'échantillon à tester à différentes concentrations (200 µL), ainsi qu'un "contrôle" (tampon phosphate, pH 6,5), sont incubés pendant 30 minutes à 37°C en présence de tyrosinase (enzyme EC 1.14.18,1, issue de champignon ; 200µL, 300 U/mL).

Mélange B : 200 µL de L-dopa (2,5 mM) sont additionnés à 400 µL de tampon phosphate (pH 6,5).

Après les 30 minutes d'incubation, le *mélange A* est ajouté au *mélange B* et la dopachrome produite est mesurée à 475 nm au bout d'une minute trente.

Les résultats obtenus sont donnés dans le tableau suivant:

| **Bloc** | **CI₅₀** |
|---|---|
| **I** | 16,0 + 1,0 µM (n=4) |
| **II** | 61,4 + 3,2 µM (n=5) |
| **III** | 5,5 + 1,2 µM (n=5) |
| **IV** | 5,4 4 ± 1, 1 µM ( n=10 ) |
| **V** | 14,4 ± 0,3 µM (n=3) |
| **VI** | 7,4 ± 0,7 µM (n=5) |
| **VII** | 13, 4 ± 0,6 µM (n=4) |
| **VIII** | 10,4 + 2,1 µM (n=8) |
| **IX** | 14 , 6 + 2,2 µM (n=4) |
| **X** | 7, 9 + 0, 9 µM (n=3) |

On constate que les fractions ou blocs de l'invention présentent une CI₅₀ inférieure à 17µM, et même pour certains inférieure à 5µM. On observera avec intérêt l'effet synergique obtenu lorsque les oligomères sont en mélange, notamment avec le resvératrol.

Le dimère ε-viniférine seul présente une IC₅₀ inférieure à 10 fois celle du monomère.

### Exemple 2 : Compositions cosmétiques

Formule N°1 (concentration en % p/p) :

| | | | |
|---|---|---|---|
| A/ | Eau désionisée (Aqua) | QSP | 100 |
| | EDTA tétrasodique | | 0,10 |
| | Glycérine | | 5,00 |
| | Butylène glycol | | 4,00 |
| | Gomme de xanthane | | 0,50 |
| | | | |
| B/ | Stéarate de glycérol | | |
| | Stéarate PEG-100 (Arlacel 165 : Uniqema) | | 5,00 |
| | Méthoxycinnamate d'éthylhexyle | | 7,50 |
| | Salicylate d'octyle | | 5,00 |
| | Dioxyde de titane | | 5,00 |
| | Triglycéride caprylique/caprique | | 8,00 |
| | Alcool cétylique | | 2,00 |
| | Diméthicone | | 0,50 |
| | | | |
| C) | Cetyl phosphate de potassium | | 1,00 |
| | | | |
| D/ | Bloc I ou bloc III à VIII Eau désionisée (Aqua) | | 0,1 à 5 1,00 |
| | | | |
| E/ | Phénoxyéthanol & Méthylparaben & Ethylparaben & Propylparaben & Butylparaben (Phenova : Croda) | | 0,80 |
| | Bisabolol | | 0,50 |
| | Arômes | | 0,50 |

### Préparations

Les mélanges A et B sont préparés séparément, chauffés à 75°C et mélangés jusqu'à dissolution complète. On ajoute ensuite :
- le mélange B au mélange A à 75°C, puis
- le mélange C au mélange AB à 75°C, et
- les mélanges D et E au mélange ABC à 30°C.

La préparation obtenue est mélangée durant 20 minutes jusqu'à l'obtention d'une crème lisse et brillante.

Formule N°2 (concentration en % p/p) :

| | | | |
|---|---|---|---|
| A/ | Eau désionisée (Aqua) | QSP | 100 |
| | EDTA tétrasodique | | 0,10 |
| | Glycérine | | 10,00 |
| | Hydroxyde de sodium | | 0,25 |
| | | | |
| B/ | Méthoxycinnamate d'éthylhexyle | | 7,50 |
| | Salicylate d'octyle | | 5,00 |
| | Benzophénone-3 | | 3,00 |
| | Butyl Méthoxydibenzoylméthane | 3,00 | |
| | Triglycéride caprylique/caprique | | 8,00 |
| | Alcool stéarylique | | 2,00 |
| | Diméthicone | | 0,50 |
| | Acide stéarique | | 4,00 |
| | Stéarate de glycérol | | 3,00 |
| | | | |
| C) | Cétyl phosphate de potassium | | 1,00 |
| | | | |
| D/ | Bloc I ou bloc III à VIII Eau désionisée (Aqua) | | 0,1 à 5 1,00 |
| | | | |
| E/ | Phénoxyéthanol & Méthylparaben | | |
| | & Ethylparaben & Propylparaben & Butylparaben (Phenova : Croda) | | 0,80 |
| | Bisabolol | | 0,50 |
| | Arômes | | 0,50 |

### Préparation

On opère comme décrit pour la préparation de la formule 1.

Formule N°3 (concentration en % p/p) :

| | | | |
|---|---|---|---|
| A/ | Eau désionisée (Aqua) | QSP | 100 |
| | EDTA tétrasodique | | 0,10 |
| | Butylène glycol | | 10,00 |
| | Gomme de xanthane | | 0,20 |
| | | | |
| B/ | Bloc I ou bloc III à VIII Eau désionisée (Aqua) | | 0,1 à 5 1,00 |
| | | | |
| C/ | Phénoxyéthanol & Méthylparaben & Ethylparaben & Propylparaben & Butylparaben | | |
| | (Phenova : Croda) | | 0,80 |
| | Bisabolol | | 0,50 |
| | Arômes | | 0,50 |
| | | | |
| D/ | Polyacrylamide & Isoparaffine en C13-14 & Laureth-7 (Sepigel 305 : Seppic) | | 1,00 |

### Préparation

On mélange la phase A puis on ajoute
- B dans A à température ambiante,
- C dans AB à température ambiante, et
- D dans ABC à température ambiante.

On mélange la préparation obtenue 20 minutes jusqu'à l'obtention d'un gel lisse et brillant.

L'invention fournit ainsi des compositions cosmétiques dotées d'un effet de blanchiment de la peau et/ou anti-tâches au moins équivalent à celui de l'acide kojique, et présentant une stabilité améliorée.

## Revendications

1. Utilisation de polyphénol en cosmétique à des fins de blanchiment de la peau et/ou anti-tâches, **caractérisée en ce que** le polyphénol est de l'ε-viniférine, des mélanges d'oligomères et/ou de polymères de resvératrol, renfermant le cas échéant du resvératrol, comprenant de l'ε-viniférine ; et leurs dérivés de type esters et éthers.

2. Utilisation selon la revendication 1, **caractérisée en ce que** l'ε-viniférine est en mélange avec du resvératrol monomère selon un rapport de 90/10 à 10/90.

3. Utilisation selon la revendication 1 ou 2, **caractérisée en ce que** ledit mélange comprend en outre des composés polyphénoliques stilbéniques et/ou flavonoïdiques.

4. Utilisation selon la revendication 3, **caractérisée en ce que** les composés flavonoïdiques sont des flavanols.

5. Utilisation selon l'une quelconque des revendications 1 à 4, pour préparer des compositions cosmétiques renfermant de 0,1 à 5% de polyphénols par rapport au poids total d'une composition cosmétique en association avec un véhicule acceptable en cosmétologie.

6. Utilisation selon la revendication 5, **caractérisée en ce que** les dites compositions cosmétiques se présentent notamment sous forme de crème, pommade, évulsion, liposome, gel, lotion, spray.

## Claims

1. A use of a polyphenol in cosmetics for skin whitening and/or stain removal purposes, **characterised in that** the polyphenol is ε-viniferin, resveratrol oligomers and/or polymers mixtures, optionally containing resveratrol, comprising ε-viniferin; and derivatives thereof of the ester and ether types.

2. The use according to claim 1, **characterised in that** ε-viniferin is in admixture with monomeric resveratrol in a ratio from 90/10 to 10/90.

3. The use according to claim 1 or 2, **characterised in that** said admixture further comprises stilbenic and/or flavonoidic polyphenolic compounds.

4. The use according to claim 3, **characterised in that** the flavonoidic compounds are flavanols.

5. The use according to anyone of claims 1 to 4, for preparing cosmetic compositions containing from 0.1 to 5% of polyphenols with respect to the total weight of a cosmetic composition in association with a cosmetically acceptable vehicle.

6. The use according to claim 5, **characterised in that** said cosmetic compositions are in particular in the form of a cream, ointment, emulsion, liposome, gel, lotion, spray.

## Patentansprüche

1. Verwendung von Polyphenol in der Kosmetik zur Bleichung der Haut und/oder gegen Pigmentflecken, **dadurch gekennzeichnet, dass** es sich bei dem Polyphenol um ε-Viniferin, Gemische von Oligomeren und/oder Polymeren von Resveratrol, die gegebenenfalls Resveratrol umfassen und die ε-Viniferin umfassen, sowie deren Derivate vom Ester- und Ethertyp handelt.

2. Verwendung gemäß Anspruch 1, **dadurch gekennzeichnet, dass** das ε-Viniferin im Gemisch mit monomerem Resveratrol im Verhältnis von 90/10 bis 10/90 vorliegt.

3. Verwendung gemäß Anspruch 1 oder 2, **dadurch gekennzeichnet, dass** das Gemisch weiterhin Polyphenolverbindungen des Stilben- und/oder Flavonoid-Typs umfasst.

4. Verwendung gemäß Anspruch 3, **dadurch gekennzeichnet, dass** es sich bei den Verbindungen des Flavonoid-Typs um Flavanole handelt.

5. Verwendung gemäß einem der Ansprüche 1 bis 4 zur Herstellung von Kosmetikzusammensetzungen, die 0,1 bis 5% Polyphenole, bezogen auf das Gesamtgewicht einer Kosmetikzusammensetzung, in Verbindung mit einem kosmetisch annehmbaren Träger umfassen.

6. Verwendung gemäß Anspruch 5, **dadurch gekennzeichnet, dass** die Kosmetikzusammensetzungen insbesondere als Creme, Pomade, Emulsion, Liposomen, Gel, Lotion, Spray vorliegen.
